# EUROPEAN PATENT APPLICATION

(11) **EP 3 827 785 A1**
(43) Date of publication of application: **02.06.2021**
(21) Application number: 19212595.3
(22) Date of filing: 29.11.2019
(51) Int. Cl.: A61F 2/24, A61F 2/95

(54) **ANNULOPLASTY DEVICE**

(71) Applicant: Medtentia International Ltd Oy, 02600 Espoo (FI)
(72) Inventor: Keränen, Olli, 23741 Bjärred (SE)
(74) Representative: Invent Horizon IP

(57) **Abstract**

An annuloplasty device is disclosed comprising first and second support rings having a coiled configuration in which the first and second support rings are arranged as a coil around a central axis, wherein the first and second support rings are configured to be arranged on opposite sides of native heart valve leaflets of a heart valve, the first and second support rings have respective free ends, a line attached at a proximal connection at a proximal tip of a first end of one of the free ends. A method of retrieving an annuloplasty device from an implantation site at a heart valve is disclosed.

## Description

### Technical field

This invention pertains in general to the field of cardiac valve replacement and repair. More particularly the invention relates to an annuloplasty device for delivery and implantation at a cardiac valve, and a method for retrieval of such annuloplasty device.

### Background

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty device, such as an annuloplasty ring, to reduce the diameter of the annulus, or modify the geometry of the annulus in any other way, or aid as a generally supporting structure during the valve replacement or repair procedure. Such annuloplasty devices are implanted into position by various delivery devices.

A problem with prior art devices are less-than-optimal engagement mechanisms between the annuloplasty device and the delivery device which do not provide sufficient reliability during the positioning phase, or during a possible repositioning to get the annuloplasty device in the final secured position, which may lead to a more complicated and time consuming procedure. Previous devices thus requre exact, i.e. time consuming, navigation and manipulation to minimize the risk of sub-optimal fixation of the annuloplasty device. During heart surgery, a premium is placed on reducing the amount of time used to replace and repair valves as the heart is frequently arrested and without perfusion. The above problems may have dire consequences for the patient and the health care system. Patient risk is increased. A problem with prior art devices seeking to improve upon the mentioned disadvantages is increased complexity of the engagement mechanisms between the annuloplasty device and the delivery device. This may lead to less cost-effectiveness, and/or bulky profiles of such devices, which typically has a negative impact on the steerability of the annuloplasty device, as well as an overall more cumbersome navigation of the annuloplasty device to its final position.

Hence, an improved annuloplasty device for delivery and implantation at a cardiac valve would be advantageous and in particular allowing for avoiding more of the above mentioned problems and compromises, and in particular allowing for a more secure implantation procedure and reducing the time of lengthy surgery procedures, and increased patient safety.

### Summary of the Invention

Accordingly, examples of the present invention preferably seek to mitigate, alleviate or eliminate one or more deficiencies, disadvantages or issues in the art, such as the above-identified, singly or in any combination by providing a device according to the appended patent claims.

According to a first aspect an annuloplasty device is provided comprising first and second support rings having a coiled configuration in which the first and second support rings are arranged as a coil around a central axis, wherein the first and second support rings are configured to be arranged on opposite sides of native heart valve leaflets of a heart valve, the first and second support rings have respective free ends, a line attached at a proximal connection at a proximal tip of a first end of one of the free ends.

According to a second aspect a method of retrieving an annuloplasty device from an implantation site at a heart valve is provided, the annuloplasty device comprising a line attached at a proximal connection at a proximal tip of a free end of the annuloplasty device, the method comprising arranging the line in a lumen of a delivery device, advancing the delivery device over the line in a direction towards the proximal tip, engaging the proximal tip with a distal portion of the delivery device, and retrieving at least part of the annuloplasty device through an opening of the distal portion and into the lumen.

Further examples of the invention are defined in the dependent claims, wherein features for the second and subsequent aspects of the disclosure are as for the first aspect mutatis mutandis.

Some examples of the disclosure provide for an annuloplasty device which can be implanted more securely.

Some examples of the disclosure provide for an annuloplasty device which is easier to reposition during an implantation procedure.

Some examples of the disclosure provide for a less time consuming positioning and fixation of an annuloplasty device at a cardiac valve.

Some examples of the disclosure provide for a less cumbersome attachment and detachment of an annuloplasty device to a delivery device.

Some examples of the disclosure provide for increased accuracy in positioning an annuloplasty device at the annulus of a cardiac valve and thereby reducing the risk of complications.

Some examples of the disclosure provide for facilitated retrieval of an annuloplasty device after being positioned at an implantation site.

Some examples of the disclosure provide for a reduced risk of damaging the cardiac valve during a repair or replacement procedure.

Some examples of the disclosure provide for increased steerability or maneuverability of the annuloplasty device.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which examples of the invention are capable of will be apparent and elucidated from the following description of embodiments of the present invention, reference being made to the accompanying drawings, in which
Fig. 1 is a schematic illustration, in a side view, of an annuloplasty device according to one example;
Fig. 2 is a schematic illustration, in a cross-sectional side view, of a detail of an annuloplasty device according to one example;
Fig. 3a is a schematic illustration, in a cross-sectional side view, of a detail of an annuloplasty device according to one example;
Fig. 3b-c are schematic illustrations of a line for connecting to an annuloplasty device according to examples of the disclosure;
Fig. 4a is a schematic illustration, in a cross-sectional side view, of a detail of an annuloplasty device according to one example;
Fig. 4b is a schematic illustration, in a cross-sectional side view, of a detail of an annuloplasty device according to one example;
Fig. 5 is a schematic illustration, in a cross-sectional side view, of a detail of an annuloplasty device according to one example;
Fig. 6 is a schematic illustration, in a cross-sectional side view, of a detail of an annuloplasty device according to one example;
Fig. 7 is a schematic illustration, in a cross-sectional side view, of a detail of an annuloplasty device according to one example;
Fig. 8 is a schematic illustration of an annuloplasty device, in a side view, where the annuloplasty device is positioned above and below valve leaflets, according to an example of the disclosure;
Figs. 9a-b are schematic illustrations of an annuloplasty device being arranged outside a delivery device (Fig. 9a) and inside a lumen of a delivery device (Fig. 9b), respectively;
Figs. 10a-b are schematic illustrations of an annuloplasty device being anchored to tissue at an implantation site (Fig. 10a) and retrieved into a lumen of a delivery device from the implantation site (Fig. 10b); and
Figs. 11a-b are flow charts of a method of retrieving an annuloplasty device from an implantation site at a heart valve, according to examples of the disclosure.

### Detailed description

Specific examples of the invention will now be described with reference to the accompanying drawings. This invention may, however, be embodied in many different forms and should not be construed as limited to the examples set forth herein; rather, these examples are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on examples applicable to cardiac valve implants such as annuloplasty rings. However, it will be appreciated that the invention is not limited to this application but may be applied to many other annuloplasty implants and cardiac valve implants including for example replacement valves, and other medical implantable devices.

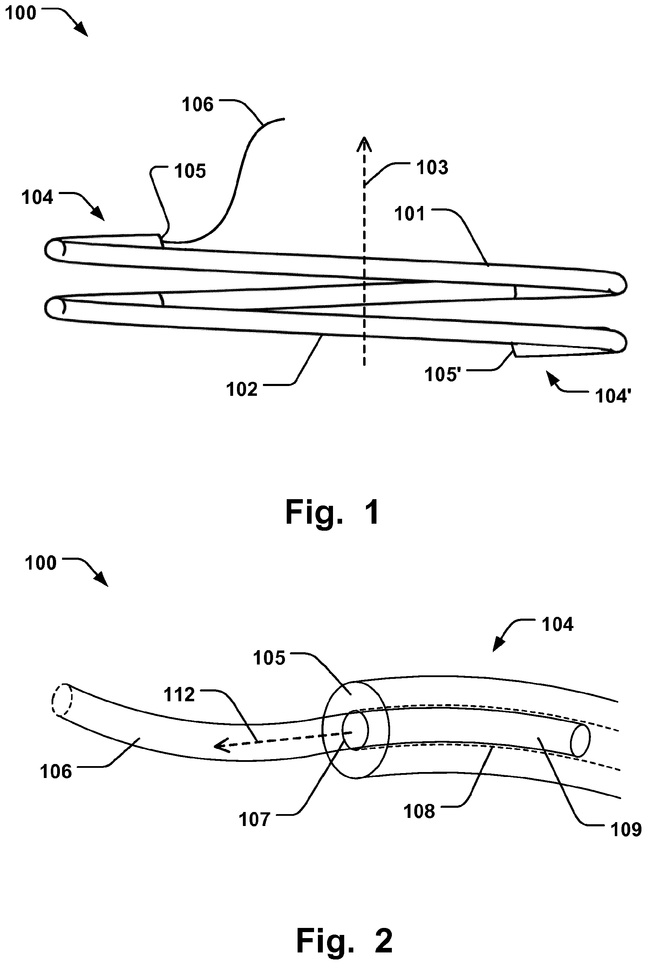

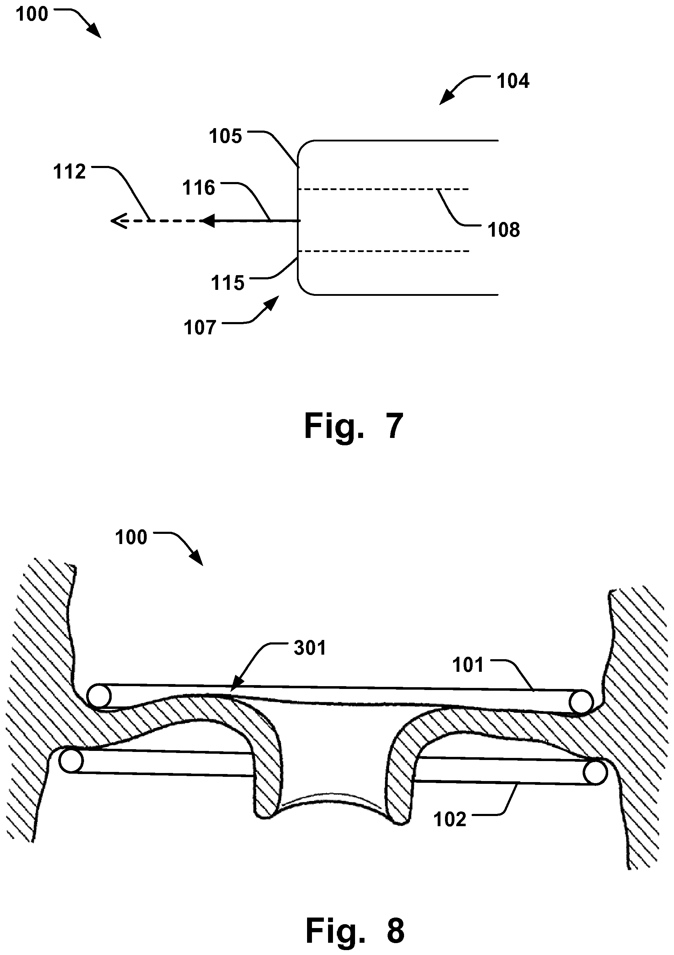

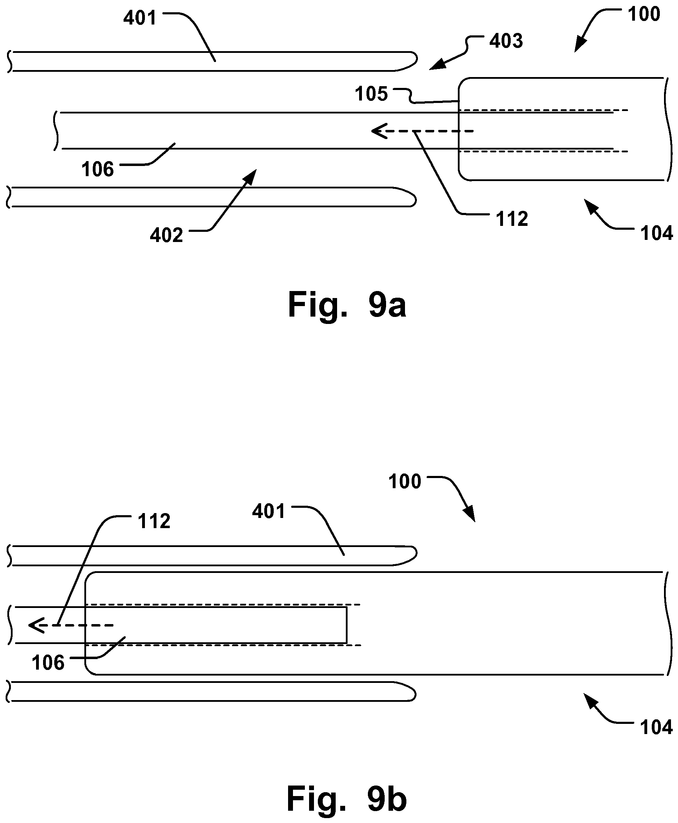

Fig. 1 schematically illustrates an example of an annuloplasty device 100 comprising a first support ring 101 and second support ring 102 which are adapted to be arranged as a coil, i.e. in a helix-shape, in a coiled configuration around a central axis 103, as illustrated in Fig. 1. The annuloplasty device 100 is arranged in the coiled configuration at least when in a relaxed state of the material from which the annuloplasty device 100 is formed, i.e. free from outside forces acting upon the annuloplasty device 100. The coil-shaped annuloplasty device 100 has two free ends 104, 104'. The first and second support rings 101, 102, and the respective free ends 104, 104', are configured to be arranged on opposite sides of native heart valve leaflets 301 of a heart valve, as illustrated in the side view of Fig. 8. As shown in Fig. 8, the first support ring 101 may be arranged on an atrial side of the heart valve, around the annulus of the valve, and the second support ring 102 may be arranged on a ventricular side. The first and second support rings 101, 102, are connected to form a coil- or helix shaped ring. The coil extends through the valve opening at a commissure thereof. The first and second support rings 101, 102, may thus assume the coiled configuration also when in an implanted state. The annuloplasty device 100 may comprise a shape-memory material, so that the annuloplasty device 100 re-assumes the coiled configuration after having been delivered from a delivery device 401, such as a delivery catheter 401 schematically shown in Figs. 9a-b, to the target site, after having been temporarily restrained in an elongated configuration inside such catheter 401. The annuloplasty device 100, i.e. annuloplasty implant 100, may comprise a shape memory material, such as NiTiNol, or another suitable biocompatible alloy that can be heat-set in defined shapes, i.e. in a defined relaxed shape in absence of outside acting forces, such as in a coiled configuration, in a heat treatment procedure.

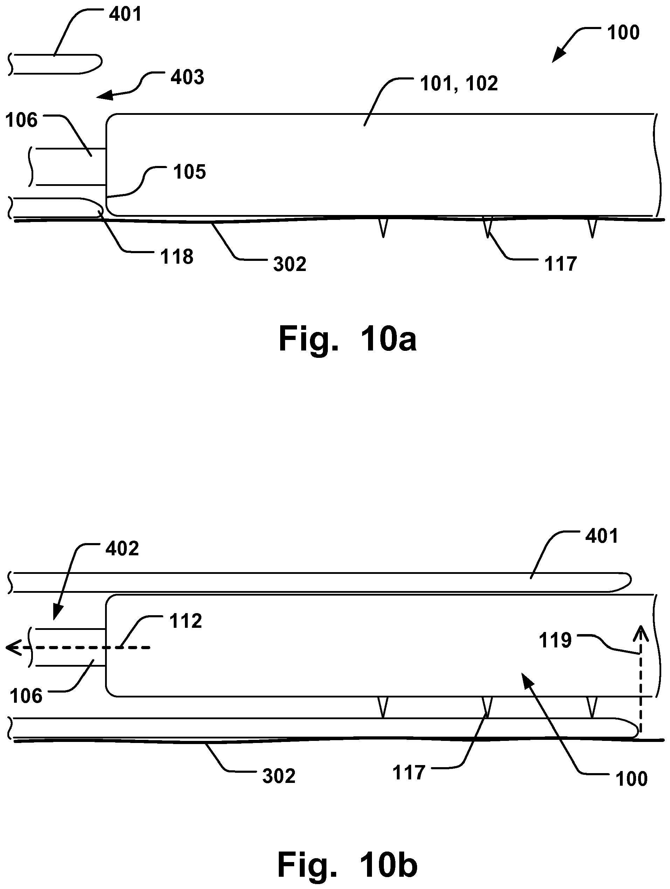

The annuloplasty device 100 comprises a line 106 attached at a proximal connection 107 at a proximal tip 105, 105', of a first end of one of the free ends 104, 104'. The line may 106 may be a suture, wire, or any other essentially flexible and elongated element sized to be arranged in, and/or connect to, a delivery device 401, as schematically shown in Figs. 9 - 10. Figs. 1 and 2 show schematic examples where a line 106 is attached at a proximal tip 105 of a free end 104, of the first support ring 101. The examples illustrated in Figs. 1 - 7, 9 - 10, show the line 106 attached to the first support ring 101, but it should be understood that the line 106 may be attached to the tip 105' of a free end 104' of the second support ring 102, although reference is made to free end 104, and proximal tip 105 throughout the present disclosure. The line 106 being attached at a proximal connection 107 at a proximal tip 105 should be construed as a line 106 being securely fixed to the proximal tip 105 such that, in use, pulling the line 106 towards a delivery device 401, such as a catheter 401, allows the first and second support rings 101, 102, to be withdraw towards such catheter 401, and thus allowing for retrieval of the annuloplasty device 100 after being previously deployed from such delivery catheter. Fig. 9a shows the annuloplasty device 100 positioned outside the delivery device 401, while Fig. 9b is a schematic illustration showing the annuloplasty device 100 being partly arranged inside a lumen 402 of the delivery device 401, e.g. if the previously deployed annuloplasty device 100 has been pulled into the lumen 402 along an axial direction 112. Fig. 10a is another schematic illustration showing a deployed annuloplasty device 100 anchored to tissue 302 with retention units 117 extending from the first and/or second support rings 101, 102. In this example a delivery device 401 is advantageously advanced towards the annuloplasty device 100 so that a distal portion 118 of the delivery device 401 engages the proximal tip 105 of the annuloplasty device 100 (Fig. 10a). Continuing advancing the delivery device 401 provides for wedging the distal portion 118 between the tissue 302 and the annuloplasty device 100 so that the retention units 117 of the latter are raised from the anchored position in Fig. 10a, i.e. moving along a radial direction 119 essentially perpendicular to the axial direction 112, as schematically illustrated in Fig. 10b, while the annuloplasty device 100 is gradually retrieved into the lumen 402 of the delivery device 401. The line 106 provides for effectively guiding the delivery device 401 into the correct position so that the proximal tip 105 is retrieved into the lumen 402. The proximal portion 118 may be formed with an at least partly curved or smooth shape as schematically illustrated in e.g. Fig. 10a. This provides for engaging the tissue 302 in a non-traumatic manner while facilitating wedging the distal portion 118 to lift the annuloplasty device 100 as described above. A method 200 is described further below in relation to Figs. 9 - 10.

The first and second support rings 101, 102, and the free ends 104, 104', thereof extend generally along an axial direction 112. The proximal tip 105 of the free end 104 should be construed as the part of the free end 104, such as a surface 115 of the free end 104, which terminates the free end 104 along the axial direction 112. The proximal connection 107 fixedly attaches the line 106 to the proximal tip 105. Having a line 106 attached to the proximal tip 105 provides for a secure retrieval of the first and second support rings 101, 102, while minimizing the cross-section of the annuloplasty device 100 in a direction perpendicular to the axial direction 112, i.e. in a radial direction 113. A more secure implantation of the annuloplasty device 100 is thus provided, since repositioning is facilitated by manipulation of the line 106. The position of the first and second support rings 101, 102, at the cardiac valve may be changed by e.g. pulling of the line 106, to optimize the respective positions on the ventricular and atrial side. The line 106 also provides for fully withdrawing the first and second support rings 101, 102, from the implantation site as described above in case of complications. The minimized cross-section provided by proximal connection at the proximal tip 105 allows for an improved maneuverability and facilitated positioning of the first and second support rings 101, 102, at the valve. The compact cross-section facilitates integration with existing delivery devices, and the procedure may be completed with less components and with fewer steps. In one aspect of the invention an annuloplasty system is provided comprising the annuloplasty device 100 as described in relation to Figs. 1 - 10 and a delivery device 401 as described in relation to Figs. 9 - 10.

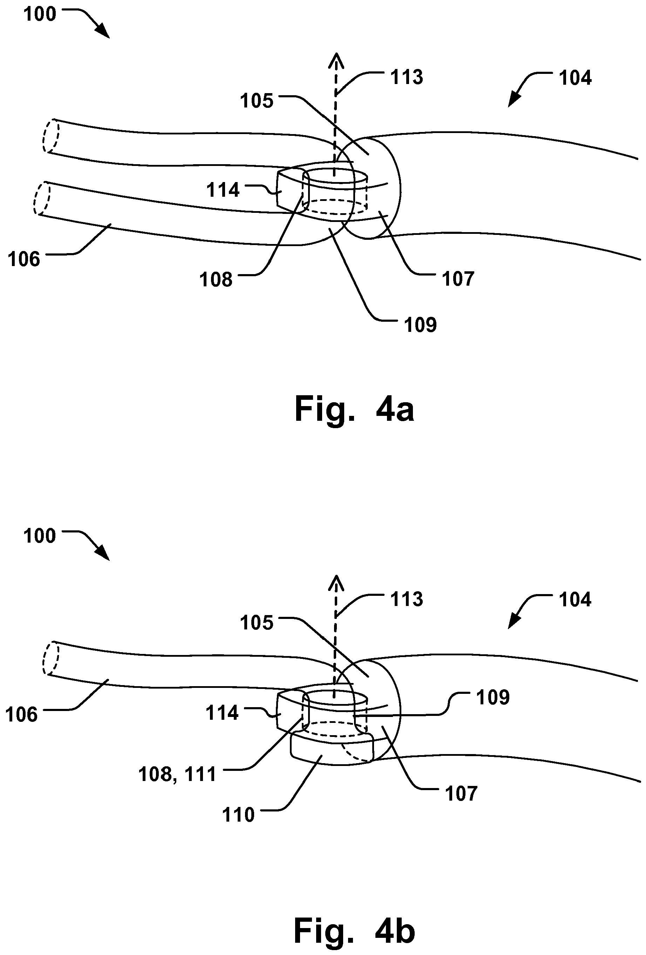

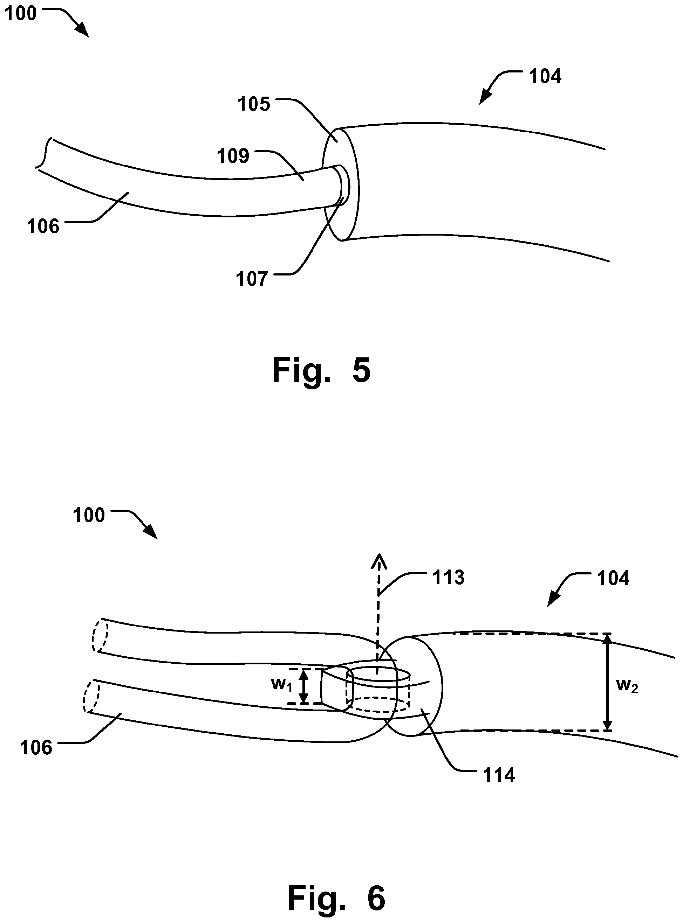

The proximal connection 107 may comprise a lumen 108 sized to receive a distal tip 109 of the line 106. Fig. 2 show a schematic example where distal tip 109 of the line 106 is arranged inside lumen 108 of the free end 104. Figs. 4a-b are further schematic examples where the proximal connection 107 comprises a lumen 108 and part of the line 106, such as a distal tip 109 thereof, is arranged in the lumen 108. Fig. 4a show an example where the line 106 is looped through lumen 108. The length of the line 106 extending past the lumen 108 may be varied, such as forming a complete looped configuration as illustrated in Fig. 4a, or extending a length through the lumen 108 corresponding essentially to the length of the lumen 108 itself, as illustrated in Fig. 4b. Having a lumen 108 arranged to receive the line 106, as exemplified in e.g. Figs. 2, 4a-b, provides for a facilitated attachment of the line 106 to the free end 104 and the proximal tip 105 thereof. In other examples it is conceivable however that the line 106 may be attached at other types of proximal connections 107. E.g. the proximal connection 107 may comprise a material or element, securing the distal tip 109 of the line 106 to the proximal tip 105, as schematically illustrated in Fig. 5, such as an adhesive material, or other material or element configured to secure the line 106, while providing for the above mentioned advantages, e.g facilitated positioning and/or retrieval of the annuloplasty device 100 while providing for a compact cross-section.

The first end 104 may be configured to be compressed to clamp the distal tip 109 of the line 106 inside the lumen 108. In the example of Fig. 2, the first end 104 may thus be compressed radially inwards, i.e. perpendicular to the axial direction 112, so that the inner walls of the lumen 108 are pushed against the distal tip 109 of the line 106. This provides for an effective and secure anchoring of the line 106 inside the lumen 108 and to the first end 104. The first end 104 may be compressed in a manufacturing process of the annuloplasty device 100 to fix the line 106. It is also conceivable that a user, e.g. before a procedure, may secure the line 106 to the first end 104 by inserting the distal tip 109 into the lumen 108 and subsequently compress the first end 104, e.g. by a tool configured to exert a compressive force in a radially inward direction on the first end 104. In one example, the lumen 108 shown in Figs. 4a-b extending in the radial direction 113, may also be compressed to anchor the line 106 inside the lumen 108. In another example the distal tip 109 may be secured to the lumen 108 by an adhesive.

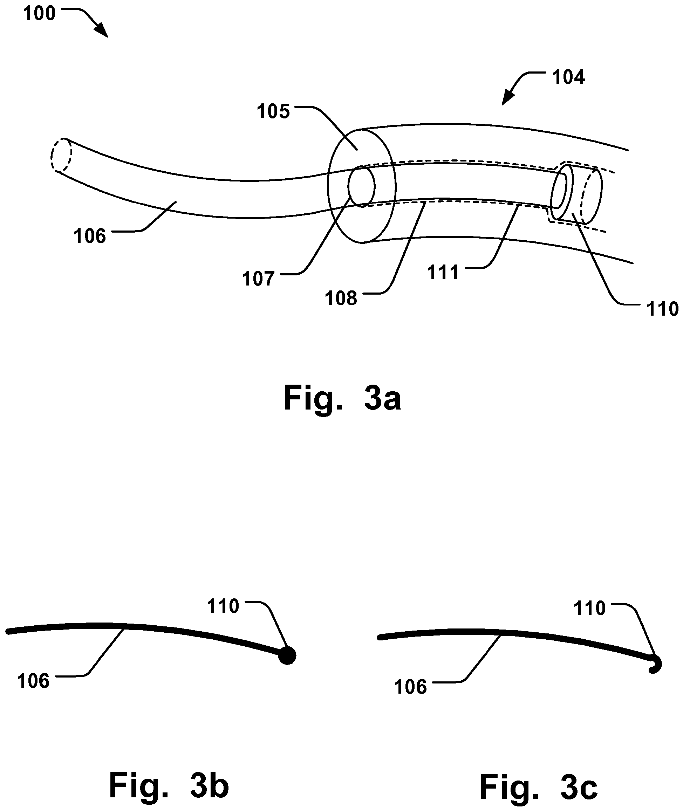

The distal tip 109 of the line 106 may comprise an expanded diameter portion 110, as schematically illustrated in Figs. 3a-c and Fig. 4. Having an expanded diameter portion 110 at the distal tip 109 provides for facilitating a secure anchoring of the line 106 to the free end 104. The expanded diameter portion 110 has an increased cross-section in the radial direction 113, i.e. a wider cross-section compared to the cross-section of the overall line 106. The expanded diameter portion 110 may have different shapes, such as cylindrical as schematically shown in Figs. 3a and 4b, or spherical as schematically illustrated in Fig. 3b, or hook-shaped as illustrated in Fig. 3c. Thus, the expanded diameter portion 110 may comprise an at least partly spherical portion or a hook-shaped portion.

The expanded diameter portion 110 may be sized to be locked against a narrow section 111 of the lumen 108, as exemplified in Fig. 3a. Fig. 4b show another example where the lumen 108 has a narrow section 111 compared to the expanded diameter portion 110, so that the latter can not be pulled through the narrow section 111. This provides for further facilitating a secure anchoring of the line 106 to the free end 104.

The lumen 108 may extend along an axial direction 112 of the first free end 104, as exemplified in Figs. 2 and 3a. This provides for an effective anchoring of the line 106 to the free end 104 as described above, since a retention force can be applied onto a readily adjustable and optimizable length the line 106. I.e. the retention force can be increased by increasing the length of the distal tip 109 extending inside the lumen 108, which may be subsequently compressed as described above to secure the distal tip 109 inside the lumen 108. A reliable anchoring of the line 106 to the free end 104 is thus provided without affecting the cross-sectional dimension of the annuloplasty device 100, i.e. the dimension in the radial direction 113. Furthermore, the need for external fixating elements may be dispensed with, which otherwise may have a negative impact on the surrounding tissue, e.g. tissue abrasion, or lead to an increase risk of formation of thrombus.

The lumen 108 may be coaxially aligned with the first end 104 along the axial direction 112. This provides for optimizing the structural integrity of the free end 104 and a more robust support ring 101. It is conceivable however that in some examples, the line 106 may be secured to a lumen 108 which is off-set in a radial direction 113 from a center of the free end 104.

The lumen 108 may comprise a drilled opening in the proximal tip 105. The lumen 108 may thus be formed in an otherwise solid support ring 101, 102. A drilled opening should be construed as any mechanism by which an opening is cut into the free end 104. A distal tip 109 of the line 106 may thus be inserted and secured to the opening or lumen as described above. In other examples, the first and/or second support ring 101, 102, may be formed from a tubular-shaped material, in which case the distal tip 109 of the line 106 may be inserted and secured to the free end 104 without the need for an extra manufacturing step of cutting an opening into the free end 104.

The lumen 108 may extend along a radial direction 113 being perpendicular to the axial direction 112, as illustrated in the examples of Figs. 4a-b. The line 106 may thus be effectively secured to the free end 104 by being inserted in the lumen 108 in the radial direction 113, which may be particularly advantageous in some applications. Securement may be provided as described above, e.g. by compressing or clamping the lumen 108 against the line 106, and/or by having an expanded diameter portion 111, and/or by other fixation mechanisms, such as an adhesive.

The proximal connection 107 may comprise a projection 114 extending from the proximal tip 105 in an axial direction 112 of the first end 104. The lumen 108 may extend through the projection 114, as schematically illustrated in Figs. 4a-b. Having a projection 114 from the proximal tip 105 to which the line 106 is secured provides for a facilitated fixation of the line 106 to the free end 104. The projection 114 may be formed from the material from which the first and/or second support ring 101, 102, is formed, or may comprise any other biocompatible material, such as stainless steel or NiTinol. The projection 114 may be integrated with the free end 104. The projection 114 may be a separate element fixed to the free end 104 or be formed as an integral extension of the free end 104, i.e. as a continuous piece of material.

The projection 114 may have a width (w₁) in the radial direction 113 which is smaller than a width (w₂) of the proximal tip 105 in the radial direction 113, as schematically illustrated in Fig.6. This provides for reducing the cross-section in the radial direction 113 since the space above and/or below the projection 114 in the radial direction 113 may accommodate at least part of the line 106, e.g. when the line 106 is folded as indicated in Fig. 6 or Fig. 4b. A more compact annuloplasty device 100 may thus be provided. Reducing the extension of the line 106 in the radial direction 113 also provides for minimizing the risk of having too high friction between the line 106 and a surrounding delivery catheter. This provides for facilitated delivery and/or retrieval of the annuloplasty device 100.

The proximal connection 107 may be arranged at least partly at a surface 115 of the proximal tip 105. The surface 115 has a normal direction 116, as schematically illustrated in Fig. 7. The surface 115 may be angled such that a vector component of the normal direction 116 is parallel with an axial direction 112 of the first end 104. In the example in Fig. 7 the normal 116 of the surface 115 is essentially parallel with the axial direction 112. I.e. the aforementioned vector component is thus equal to the normal 116. It is however conceivable that the normal 116 of the surface 115 may have an angle other than zero relative the axial direction 112, i.e. an acute angle, so that at least a vector component of the normal 116 is parallel with the axial direction 112. Having the proximal connection 107 at such surface 115 of the proximal tip 105 provides for maintaining a minimized cross-section in the radial direction 113. I.e. having the line 106 attached to such surface 115, or proximal connection 107, limits the cross-sectional width compared to a case where a line, such as a suture, would be attached to other sections of the first and/or second support rings 101, 102, such that the suture would extend from the surface of the first and/or second support ring 101, 102, itself, i.e. anywhere between the free ends 104', 104. In comparison, the proximal attachment, to the surface 115, as described above does not add to the width of the annuloplasty device 100 in the radial direction 113, or at least limits any contribution to the width in the radial direction 113. This further provides for a compact annuloplasty device 100, which is easier to navigate to a target location.

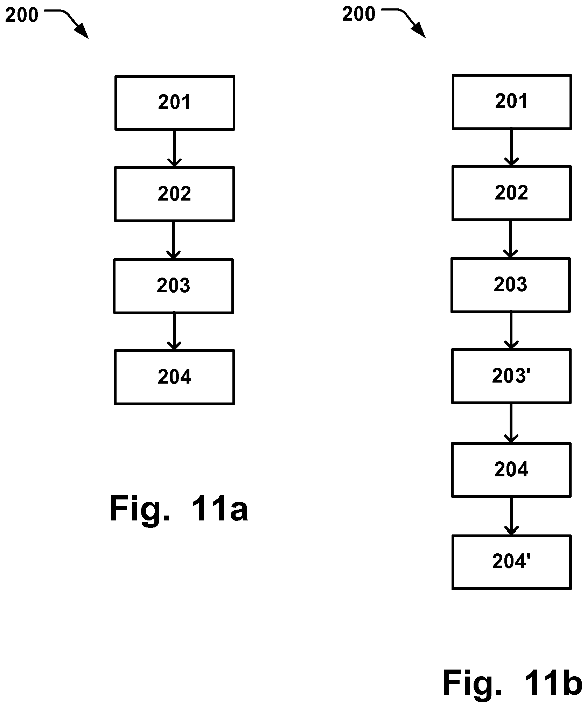

Fig. 11a in conjunction with Figs. 9 - 10 shows a flowchart of a method 200 of retrieving an annuloplasty device 100 from an implantation site at a heart valve. The annuloplasty device 100 comprises a line 106 attached at a proximal connection 107 at a proximal tip 105, 105' of a free end 104, 104' of the annuloplasty device 100, as described above. The method 200 comprises arranging 201 the line 106 in a lumen 402 of a delivery device 401, and advancing 202 the delivery device 401 over the line 106 in a direction towards the proximal tip 105, 105'. The method 200 comprises engaging 203 the proximal tip 105, 105', with a distal portion 118 of the delivery device 401, and retrieving 204 at least part of the annuloplasty device 100, i.e. the first and/or second support ring 101, 102, through an opening 403 of the distal portion 118 and into the lumen 402 (Figs. 9b and 10b). As elucidated above, the line 106 provides for an effective guiding of the delivery device 401 into the correct position so that the proximal tip 105 is retrieved into the lumen 402. The procedure may thus be completed in a reduced amount of time, and facilitates both partly retrieval for repositioning and complete retrieval and removal of the annuloplasty device 100 from the implantation site.

Fig. 11b in conjunction with Figs. 9 - 10 shows another flowchart of a method 200 of retrieving an annuloplasty device 100 from an implantation site at a heart valve. The method 200 may comprise wedging 203' the distal portion 118 between tissue 302 at the implantation site and the annuloplasty device 100 so that retention units 117 which in this example are fixed to the annuloplasty device 100 are raised from an anchored position (Fig. 10a) in the tissue and moved in a radial direction 119 (Fig. 10b) essentially perpendicular to an axial direction 112 along which the annuloplasty device 100 extends with an elongated shape. The retention units 117 may thus be lifted from their anchored positions as the delivery device 401 is gradually advanced over the annuloplasty device 100. The method 200 may thus comprise retrieving 204' the annuloplasty device 100 and its retention units 117 into the lumen 402. The line 106 provides for an effective guiding of the delivery device 401 into the correct position so that the proximal tip 105 is retrieved into the lumen 402, and subsequent disengagement of the retention units 117 from the tissue. This provides for an effective and facilitated retrieval of an annuloplasty device 100 which is anchored to tissue with retention units 117.

The present invention has been described above with reference to specific embodiments. However, other embodiments than the above described are equally possible within the scope of the invention. The different features and steps of the invention may be combined in other combinations than those described. The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. An annuloplasty device (100) comprising
first (101) and second (102) support rings having a coiled configuration in which the first and second support rings are arranged as a coil around a central axis (103),
wherein the first and second support rings are configured to be arranged on opposite sides of native heart valve leaflets (301) of a heart valve,
the first and second support rings have respective free ends (104, 104'),
a line (106) attached at a proximal connection (107) at a proximal tip (105, 105') of a first end of one of the free ends.

2. Annuloplasty device according to claim 1, wherein the proximal connection comprises a lumen (108) sized to receive a distal tip (109) of the line.

3. Annuloplasty device according to claim 2, wherein the first end is configured to be compressed to clamp the distal tip of the line inside the lumen.

4. Annuloplasty device according to claim 2 or 3, wherein the distal tip of the line comprises an expanded diameter portion (110).

5. Annuloplasty device according to claim 4, wherein the expanded diameter portion is sized to be locked against a narrow section (111) of the lumen.

6. Annuloplasty device according to claim 4 or 5, wherein the expanded diameter portion comprises an at least partly spherical portion or a hook-shaped portion.

7. Annuloplasty device according to any of claims 2 - 6, wherein the lumen extends along an axial direction (112) of the first end.

8. Annuloplasty device according to claim 7, wherein the lumen is coaxially aligned with the first end along the axial direction.

9. Annuloplasty device according to claim 7 or 8, wherein the lumen comprises a drilled opening in the proximal tip.

10. Annuloplasty device according to any of claims 2 - 6, wherein the lumen extends along a radial direction (113) being perpendicular to the axial direction.

11. Annuloplasty device according to claim 10, wherein the proximal connection comprises a projection (114) extending from the proximal tip in an axial direction (112) of the first end, wherein the lumen extends through the projection.

12. Annuloplasty device according to claim 11, wherein the projection has a width (w₁) in the radial direction smaller than a width (w₂) of the proximal tip in the radial direction.

13. Annuloplasty device according to any of claims 1 - 12, wherein the proximal connection is arranged at least partly at a surface (115) of the proximal tip, the surface having a normal direction (116), the surface being angled such that a vector component of the normal direction is parallel with an axial direction (112) of the first end.

14. Method (200) of retrieving an annuloplasty device (100) from an implantation site at a heart valve, the annuloplasty device comprising a line (106) attached at a proximal connection (107) at a proximal tip (105, 105') of a free end (104, 104') of the annuloplasty device, the method comprising
arranging (201) the line in a lumen (402) of a delivery device (401),
advancing (202) the delivery device over the line in a direction towards the proximal tip,
engaging (203) the proximal tip with a distal portion (118) of the delivery device, and
retrieving (204) at least part of the annuloplasty device through an opening (403) of the distal portion and into the lumen.

15. Method according to claim 14, comprising
wedging (203') the distal portion between tissue (302) at the implantation site and the annuloplasty device so that retention units (117) attached to the annuloplasty device are raised from an anchored position in the tissue and moved in a radial direction (119) essentially perpendicular to an axial direction (112) along which the annuloplasty device extends with an elongated shape,
retrieving (204') the annuloplasty device and its retention units into the lumen.
